# EUROPÄISCHE PATENTSCHRIFT

(11) **EP 1 114 630 B1**
(45) Veröffentlichungstag und Bekanntmachung des Hinweises auf die Patenterteilung: **18.05.2005**
(21) Anmeldenummer: 00128750.7
(22) Anmeldetag: 30.12.2000
(51) Int. Cl.: A61F 13/06, D04B 21/14

(54) **Kniebandage**
Knee bandage
Genouillère

(30) Priorität: 07.01.2000 DE 20000265 U; 16.03.2000 DE 10012984
(43) Veröffentlichungstag der Anmeldung: 11.07.2001
(73) Patentinhaber: Otto Bock HealthCare GmbH, 37115 Duderstadt (DE)
(72) Erfinder: Schlomski, Jens, 37115 Duderstadt (DE)
(74) Vertreter: Rehmann, Thorsten, Dipl.-Ing.

(56) Entgegenhaltungen:
- GB-A- 2 127 697
- US-A- 5 474 524
- US-A- 5 925 010

## Beschreibung

Die Erfindung betrifft eine Gelenkbandage zum Überziehen, bestehend aus einem elastischen Schlauch mit einer Vorderseite und einer dieser gegenüberliegenden Beugeseite.

Eine solche Bandage ist beispielsweise aus der US-A-5,474,524 bekannt und kann zur Stützung der menschlichen Gelenke, z.B. des Ellbogens oder Knies, verwendet werden. Kniegelenkbandagen bzw. -orthesen werden beispielsweise zur Stützung instabiler Kniegelenke während der konservativen postoperativen Behandlung von Kreuzbandrupturen und auch zur Linderung der durch arthrotische Knie hervorgerufenen Schmerzen und zur Stützung der Knie nach Seitenbandverletzungen verwendet. Die Kniegelenkbandagen werden über das Kniegelenk des Patienten gezogen und von ihm während der Behandlung täglich aber insbesondere beim Gehen und Laufen getragen. Nachteilig bei herkömmlichen Kniegelenkbandagen ist, dass sie beim Gehen oder Laufen im Kniekehlenbereich Falten werfen und die Kniebeuge in der Beugestellung abschnüren. Die herkömmlicherweise fast vollständig aus einem Flachgestrick oder Rundgestrick hergestellten Kniegelenkbandagen führen zu einer vermehrten Schweißbildung im Kniebeugebereich des Patienten und fördern somit ein Wundscheuern des Kniebeugebereichs.

Von dieser Problemstellung ausgehend, soll eine verbesserte Gelenkbandage zum Überziehen zur Verfügung gestellt werden.

Zur Problemlösung zeichnet sich eine gattungsgemäße Gelenkbandage dadurch aus, dass zwei unterschiedliche Gewirke zur Schlauchbildung miteinander vernäht sind und das Gewirk an der Beugeseite ein über die volle Länge des Schlauches reichendes, zweilagiges feuchteleitendes Abstandsgewirk ist.

Die Gelenkbandage ist über ein Kniegelenk aber auch über ein Ellenbogengelenk oder andere Gelenke ziehbar. Die Beugeseite des elastischen Schlauches ist zur Auflage in der Gelenkbeuge und die Vorderseite des elastischen Schlauches zur Auflage auf der der Beuge abgewandten Gelenkseite bestimmt. Der Schlauch ist in Längsrichtung über die Gliedmaße des Patienten auf das zu stützende Gelenk ziehbar. Dabei ist der Schlauch vorzugsweise so ausgebildet, dass er sich unter maximalem Flächenkontakt eng an die Gliedmaße schmiegt.

Das zweilagige feuchteleitende Abstandsgewirk wirkt einer Schweißansammlung im Beugebereich durch Feuchtigkeitstransport von der Haut nach außen entgegen. Die Gewirkbindung verhindert einen starken Faltenwurf der Gelenkbandage in der Beugestellung des Gelenkes.

Dadurch, dass das Abstandsgewirk über die volle Länge des Schlauches reicht, wird die Herstellung der Gelenkbandage vereinfacht. Der Schlauch wird also aus zwei unterschiedlich breiten Gewirken gebildet, die miteinander verbunden werden. Die Verbindung erfolgt vorzugsweise über zwei Flachnähte.

Wenn das Abstandsgewirk bezogen auf die Längsrichtung in einem mittleren Abschnitt verbreitert ausgebildet ist, kann die positive Wirkung des Feuchtetransports gerade im Bereich der Gelenkbeuge des die Bandage tragenden Patienten verbessert werden.

Vorzugsweise verlaufen die beiden Lagen des Abstandsgewirks im Wesentlichen parallel zueinander und sind durch feuchteleitende Verbindungsfäden, die einen Winkel von etwa 45° zu den Lagen aufweisen, auf ca. 2 mm bis 3 mm Abstand voneinander gehalten.

Das zur Auflage in der Gelenkbeuge bestimmte Abstandsgewirk transportiert Feuchtigkeit von der auf der Haut des Patienten direkt aufliegenden körperseitigen Lage über die feuchteleitenden Verbindungsfäden zur im wesentlichen parallel zur körperseitigen Lage verlaufenden körperabseitigen Lage des Gewirkes.

In einer bevorzugten Ausführungsform der erfindungsgemäßen Gelenkbandage weist der Schlauch ein Flachgestrick auf. Das Flachgestrick kann etwa zur Hälfte Kunststofffasern (gekräuseltes Perlon- und/oder Nylongarn), zur anderen Hälfte Gummi und Elastofasern aufweisen.

Durch die Ausbildung der Gelenkbandage im Wesentlichen als Flachgestrick wird ein maximaler Flächenkontakt zwischen der Haut des Patienten und der Gelenkbandage erzeugt. Aufgrund der bevorzugten Stoffzusammensetzung des Flachgestrickes wird ein günstiges Verhältnis zwischen Elastizität zur Anpassung an die sich durch Bewegung ändernde Gelenkform und Stabilität zur Stützung des Gelenkes erreicht.

Vorzugsweise sind die Vorderseite und Beugeseite durch Flachnähte miteinander verbunden.

In einer weiteren bevorzugten Ausführungsform besteht das Abstandsgewirk aus Polyester, Polyamid und Lycra. Je nach Wahl der Gewirkbindung ist das Abstandsgewirk dem Einsatzzweck entsprechend dehnungsarm oder bestimmt elastisch gestaltet. Das Abstandsgewirk kann in Längsrichtung und quer zur Längsrichtung unterschiedlich elastisch ausgestaltet sein. Das durch diese Materialien hergestellte Abstandsgewirk zeichnet sich durch seine Pflegeleichtigkeit, Dauerhaftigkeit und seinen Tragekomfort aus.

Anhand einer Zeichnung sollen Ausführungsbeispiele der Erfindung nachfolgend näher erläutert werden. Es zeigt:
- Figur 1 -: die Vorderseite einer erfindungsgemäßen Gelenkbandage;
- Figur 2 -: die Beugeseite der Gelenkbandage nach Fig. 1;
- Figur 2a -: die Ansicht einer Variante der Gelenkbandage gemäß Fig. 2;
- Figur 3 -: eine Seitenansicht der Gelenkbandage nach Fig. 2;
- Figur 4 -: ein Detail IV des Abstandsgewirks in vergrößertem Maßstab

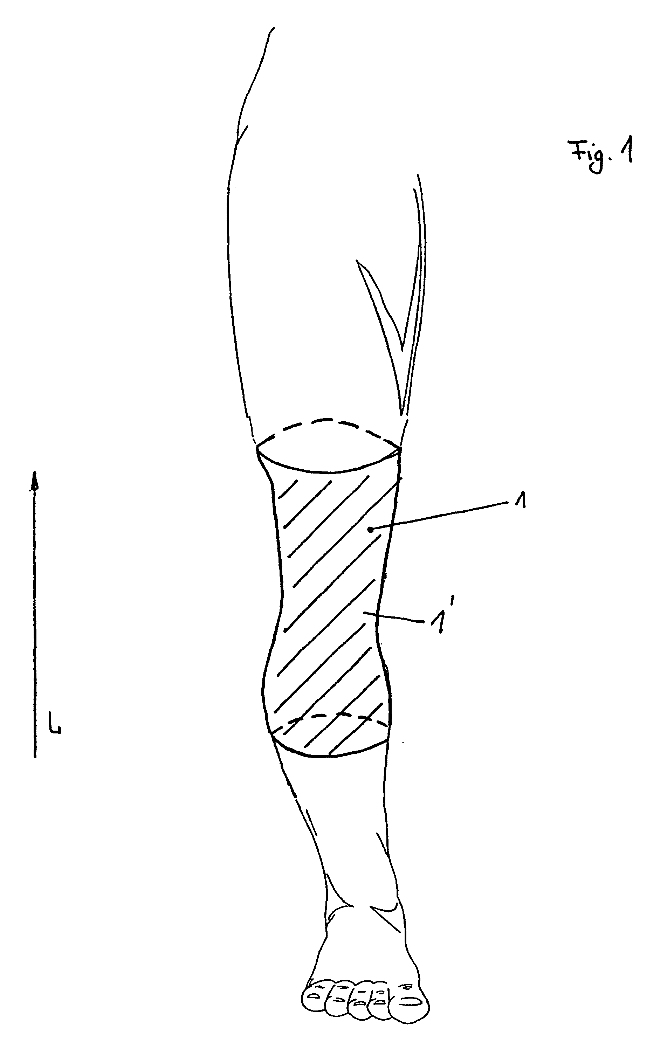

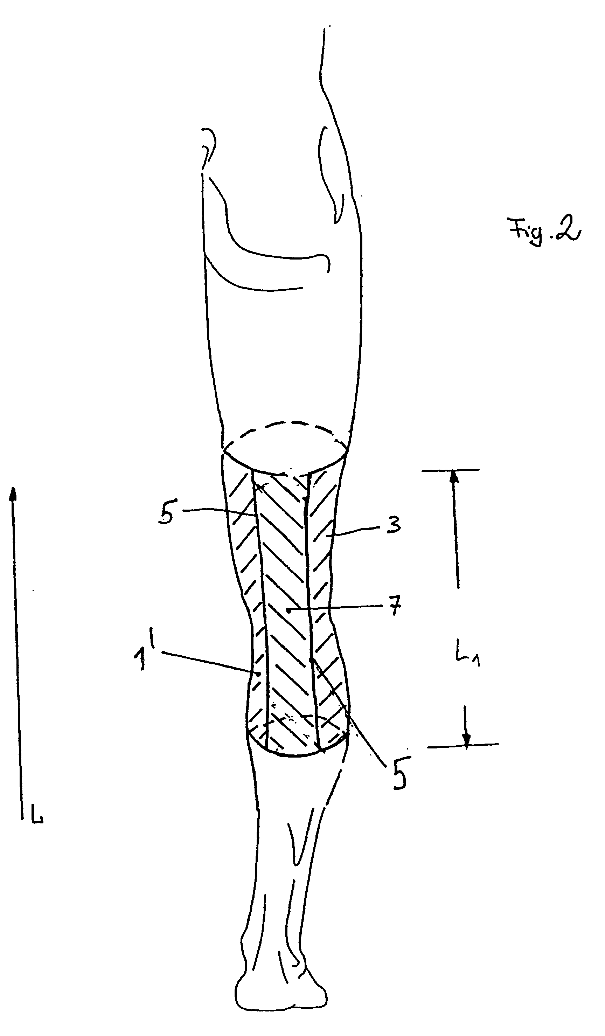

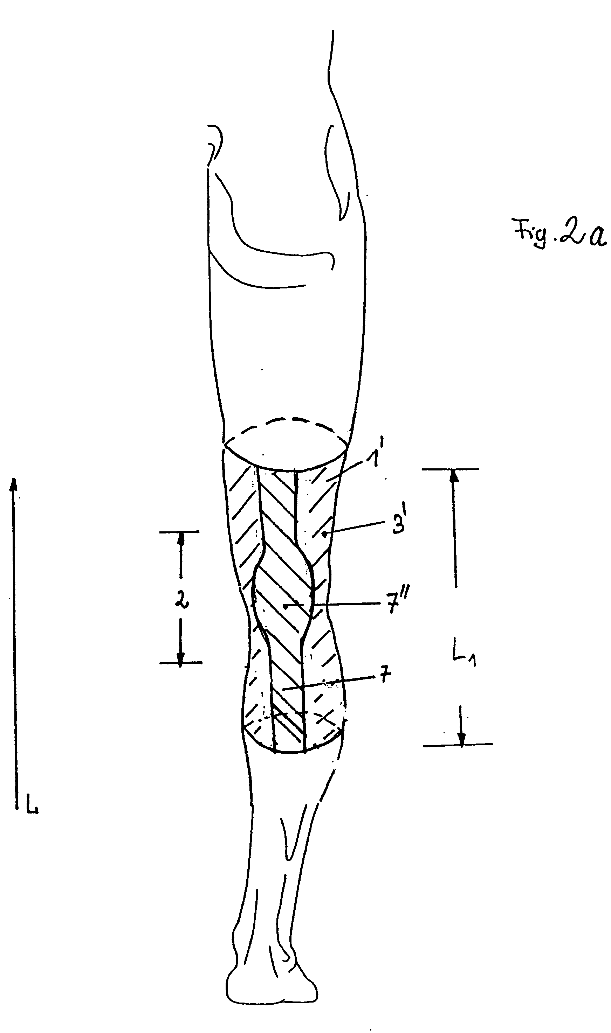

Figur 1 zeigt die Vorderseite 1 einer schlauchförmigen Gelenkbandage. Diese besteht aus einem einteiligen Flachgestrick und ist in einer Recht-Rechts-Bindung gestrickt. Die verwendeten Garne teilen sich dabei auf 50% gekräuselte Kunststofffaser (Helanca PH), 46% Gummifäden und 4% Elastofasern auf. Die Vorderseite 1 und die ihr gegenüberliegende Beugeseite 3 haben in ihrer Projektionsfläche die Form eines Rechtecks mit in einem mittleren Abschnitt 2 zueinander gekrümmten Längsseiten.

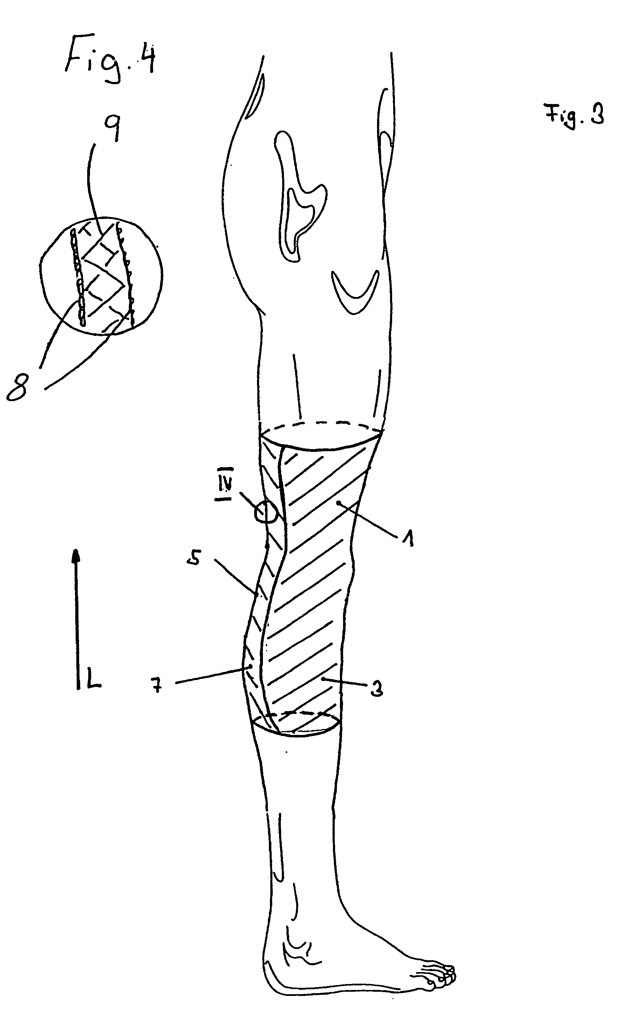

Wie aus Fig. 2 ersichtlich ist, reicht das Flachgestrick 1' bis auf die Rückseite 3 der Gelenkbandage. Über die volle Länge L₁ ist auf der Rückseite 3 ein streifenförmiges, zweilagiges und feuchteleitendes Abstandsgewirk 7 konfektioniert, das über Flachnähte 5 mit dem Flachgestrick 1' verbunden. Bei der in Figur 2a gezeigten Variante ist das Abstandsgewirk 7 in einem mittleren Abschnitt 2 verbreitert ausgebildet, wie mit Bezugsziffer 7" gekennzeichnet ist. Die Verbreiterung 7" ist so gewählt, dass sie über einen möglichst weiten Bereich der Gelenkbeuge (hier die Kniekehle) reicht, um in der kritischen Zone einen ausreichenden Feuchtetransport nach außen zu gewährleisten. Der Aufbau des Abstandsgewirks soll anhand von Fig. 4 weiter unten näher beschrieben werden.

Die beiden Lagen 8 des Abstandsgewirkes 7,7' sind auf 2 bis 3 mm Abstand voneinander gehalten (Figur 4). Die beiden Außenflächen der beiden Lagen 8 sind kleinflächig gemustert. Das Abstandsgewirk 7,7' ist bielastisch ausgebildet. Verbindungsfäden 9 zwischen den beiden zueinander parallelen Lagen 8 des Abstandsgewirks 7,7' verlaufen in einem Winkel von etwa 45° zu den Lagen 8 des Abstandsgewirks 7,7'. In den beiden Lagen 8 ist Polyamid 33 f12 und Lycra 44 dtex verarbeitet. Die Verbindungsfäden 9 weisen Polyester und ein feuchteleitendes System auf. In die beiden Schlauchöffnungen kann je ein elastisches Abschlussband eingesetzt werden.

### Bezugszeichenliste

- 1: Vorderseite
- 1': Flachgestrick
- 2: Mittlerer Abschnitt
- 3: Rückseite/Beugeseite
- 5: Flachnaht
- 6: Längsseite der Öffnung
- 7: Abstandsgewirk
- 7': Abstandsgewirk
- 7": Verbreiterung
- 8: Lagen
- 9: Verbindungsfäden
- d: Durchmesser
- L: Längsrichtung
- L₁: Länge

## Patentansprüche

1. Gelenkbandage zum Überziehen, bestehend aus einem elastischen Schlauch (1, 3) mit einer Vorderseite (1) und einer dieser gegenüberliegenden Beugeseite (3), **dadurch gekennzeichnet, dass** zwei unterschiedliche Gewirke zur Schlauchbildung miteinander vernäht sind und das Gewirk an der Beugeseite (3) ein über die volle Länge L, des Schlauches (1, 3) reichendes, zweilagiges feuchteleitendes Abstandsgewirk (7) ist.

2. Gelenkbandage nach Anspruch 1, **dadurch gekennzeichnet, dass** das Abstandsgewirk (7) bezogen auf die Längsrichtung L in einem mittleren Abschnitt (2) verbreitert ausgebildet ist.

3. Gelenkbandage nach Anspruch 1 oder 2, **dadurch gekennzeichnet, dass** der Schlauch (1, 3) ein Flachgestrick (1') aufweist.

4. Gelenkbandage nach Anspruch 3, **dadurch gekennzeichnet, dass** das Flachgestrich (1') in etwa zur Hälfte gekräuseltes Perlon- und/oder Nylongarn und zur anderen Hälfte Gummi und Elastofasern aufweist.

5. Gelenkbandage nach einem oder mehreren der vorstehenden Ansprüche, **dadurch gekennzeichnet, dass** das Abstandsgewirk (7, 7') durch Flachnähte mit dem Schlauch (1, 3) vernäht ist.

6. Gelenkbandage nach einem oder mehreren der vorstehenden Ansprüche, **dadurch gekennzeichnet, dass** die beiden Lagen (8) des Abstandsgewirks (7, 7') im Wesentlichen parallel zueinander verlaufen und durch feuchteleitende Verbindungsfäden (9), die einen Winkel von etwa 45 ° zu den Lagen (8) aufweisen, auf ca. 2 bis 3 mm Abstand voneinander gehalten werden.

7. Gelenkbandage nach einem oder mehreren der vorstehenden Ansprüche, **dadurch gekennzeichnet, dass** das Abstandsgewirk (7, 7') aus Polyester, Polyamid und Lycra besteht.

## Claims

1. Pull-on bandage for a joint, comprising an elastic hose (1, 3) with a front section (1) and with a rear section (3) located opposite the front section (1), **characterized in that** two different knitted fabrics are sewn together to form the hose, and the knitted fabric on the rear section (3) is a double-ply, moisture-conducting knitted spacer fabric (7) extending along the full length L₁ of the hose (1, 3).

2. Joint bandage according to Claim 1, **characterized in that** the knitted spacer fabric (7) is widened in relation to the longitudinal direction L in a central portion (2).

3. Joint bandage according to Claim 1 or 2, **characterized in that** the hose (1, 3) comprises a flat knitted fabric (1').

4. Joint bandage according to Claim 3, **characterized in that** approximately half of the flat knitted fabric (1') comprises crimped perlon and/or nylon yarn and the other half comprises rubber and elastomeric fibres.

5. Joint bandage according to one or more of the preceding claims, **characterized in that** the knitted spacer fabric (7, 7') is sewn to the hose (1, 3) with flat seams.

6. Joint bandage according to one or more of the preceding claims, **characterized in that** the two plies (8) of the knitted spacer fabric (7, 7') extend substantially parallel to one another and are held at a distance of ca. 2 to 3 mm from one another by moisture-conducting connection filaments (9) which are at an angle of approximately 45° with respect to the plies (8).

7. Joint bandage according to one or more of the preceding claims, **characterized in that** the knitted spacer fabric (7, 7') is made of polyester, polyamide and lycra.

## Revendications

1. Bandage articulaire de couverture, constitué d'une gaine élastique (1,3) comportant un côté antérieur (1) et à l'opposé de celui-ci un côté flexion (3), **caractérisé en ce que** deux maillages différents sont cousus l'un à l'autre pour former la gaine et le maillage côté flexion (3) est un maillage d'espacement (7,7') à deux couches, conducteur de l'humidité, s'étendant sur toute la longueur (L₁) de la gaine (1,3).

2. Bandage articulaire selon la revendication 1, **caractérisé en ce que** le maillage d'espacement (7) est réalisé élargi dans une zone médiane (2) par rapport à la direction longitudinale L

3. Bandage articulaire selon la revendication 1 ou 2, **caractérisé en ce que** la gaine (1,3) présente un tricot plat (1').

4. Bandage articulaire selon la revendication 3, **caractérisé en ce que** le tricot plat (1') présente, pour environ la moitié, du fil de nylon et/ou perlon frisé, et pour l'autre moitié du caoutchouc et des élastofibres.

5. Bandage articulaire selon l'une ou plusieurs des revendications précédentes, **caractérisé en ce que** le maillage d'espacement (7,7') est cousu avec la gaine (1,3) par des coutures plates.

6. Bandage articulaire selon l'une ou plusieurs des revendications précédentes, **caractérisé en ce que** les deux couches (8) du maillage d'espacement (7,7') s'étendent sensiblement parallèlement l'une à l'autre et sont maintenues à environ 2 à 3mm de distance l'une de l'autre par des brins de liaison (9), conducteurs de l'humidité, qui forment un angle d'environ 45° avec les couches (8).

7. Bandage articulaire selon l'une ou plusieurs des revendications précédentes, **caractérisé en ce que** le maillage d'espacement (7,7') est constitué de polyesther, polyamide, et lycra.
